# EUROPÄISCHE PATENTSCHRIFT

(11) **EP 1 749 502 B1**
(45) Veröffentlichungstag und Bekanntmachung des Hinweises auf die Patenterteilung: **02.04.2008**
(21) Anmeldenummer: 05017090.1
(22) Anmeldetag: 05.08.2005
(51) Int. Cl.: A61C 19/00, A61B 19/00

(54) **Pflege- oder Reinigungsgerät für medizinische Instrumente**
Apparatus for the maintenance and the cleaning of medical instruments
Appareil pour l'entretien et le nettoyage d'instruments médicaux

(43) Veröffentlichungstag der Anmeldung: 07.02.2007
(73) Patentinhaber: W & H Dentalwerk Bürmoos GmbH, 5111 Bürmoos (AT)
(72) Erfinder: Helfenbein, Gerald, 5133 Gilgenberg (AT); Schaffarzick, Daniel, 5061 Elsbethen (AT)

(56) Entgegenhaltungen:
- DE-A1- 10 225 232
- DE-A1- 19 913 962
- US-A- 5 733 117

## Beschreibung

Die vorliegende Erfindung betrifft ein Pflege- oder Reinigungsgerät für medizinische Instrumente sowie ein Verfahren zum Betrieb eines Pflege- oder Reinigungsgeräts.

Ein derartiges Pflege- oder Reinigungsgerät ist aus der DE 199 13 962 A1 bekannt. Das Gerät umfasst mehrere Kupplungen zum Anschluss der zu reinigenden Instrumente, wobei jeder Kupplung ein Sensor zugeordnet ist. Jede Kupplung ist des Weiteren mit einer Leitung für Reinigungsmittel verbunden, die ein ansteuerbares Ventil aufweist. Wird ein Instrument an der Kupplung angebracht, so aktiviert das Instrument den Sensor zur Abgabe eines Signals, das an eine Steuervorrichtung weitergeleitet wird. Empfängt die Steuervorrichtung dieses Signal, so öffnet sie das Ventil, das über die Leitung mit jener Kupplung verbunden ist, deren Sensor das Signal abgegeben hat. Empfängt die Steuervorrichtung kein Signal, so schließt sie das entsprechende Ventil. Auf diese Weise werden nur jene Kupplungen mit Pflege- oder Reinigungsmittel beaufschlagt, an denen ein Instrument angeschlossen ist.

Nachteil dieses Pflege- oder Reinigungsgeräts ist, dass der Sensor durch das Instrument aktiviert wird. Dies bedeutet, dass der Sensor immer in unmittelbarer Nähe zum Instrument und damit im Pflegeraum angeordnet sein muss. Da zum Reinigen der Instrumente Wasser, Dampf sowie aggressive Reinigungs- und Desinfektionsmittel verwendet werden, sind die Sensoren diesen Medien ausgesetzt und müssen entweder mit Schutzvorrichtungen versehen werden, so dass sie nicht in Kontakt mit diesen Medien geraten, oder unterliegen einem erhöhten Verschleiß-, Fehlfunktions- oder Ausfallsrisiko. Auch ist es mit diesem Pflege- oder Reinigungsgerät nicht möglich, verschiedene Instrumententypen zu unterscheiden.

Ein weiteres Pflege- oder Reinigungsgerät ist aus der DE 10 225 232 A1 bekannt. Das Gerät weist mehrere Klupplungsteile für unterschiedliche Instrumente, ein Versorgungsgerät und eine elektronische Steuereinrichtung mit einer Auswerteschaltung für die gelesenen Daten zum Bereitstellen von Plegemedien, z.B. Druckluft oder ein Pflegemittel, und Zuführen dieser Medien zum Instrument und zum Steuern dieser Maßnahmen zwecks Durchführung der Reinigung und/oder Pflege auf.

Der vorliegenden Erfindung liegt daher die Aufgabe zugrunde ein Pflege- oder Reinigungsgerät zu schaffen, das diese Nachteile nicht aufweist.

Diese Aufgabe wird gemäß der vorliegenden Erfindung durch ein Verfahren mit den Merkmalen des Anspruchs 1 und ein Pflege- oder Reinigungsgerät mit den Merkmalen des Anspruchs 6 gelöst. Besonders vorteilhafte Ausführungsformen sind in den Unteransprüchen angeführt.

Unbeschadet der unterschiedlichen Typen und des unterschiedlichen Aufbaus ist vielen medizinischen Instrumenten gemein, dass sie eine längliche, im Wesentlichen rohrförmige Außenhülse mit nur wenigen, meist schmalen und kleinen Öffnungen zur Umgebung haben. Unter Instrument sind insbesondere gerade und gebogene Handstücke für unterschiedlichste medizinische und chirurgische Anwendung zu verstehen, so zum Beispiel für dentale Anwendungen, wie das Bohren von Kavitäten, endodontische und prophylaktische Behandlungen, sowie chirurgische und orthopädische Anwendungen, wie zum Beispiel das Sägen oder Bohren von Knochen. Das Innere der Außenhülse ist mit verschiedenen Einbauten wie Motore, Wellen, Lichtquellen, Lichtleiter, Medienleitungen, Düsen, Ventile, Werkzeugkupplungen, Steuervorrichtungen, Batterien usw. befüllt. Wird ein derartiges Instrument an die Kupplungsvorrichtung eines Pflege- oder Reinigungsgeräts angeschlossen und durch die Kupplungsvorrichtung Druckluft gefördert, so muss die Druckluft durch die Außenhülse des Instruments mit ihren wenigen durch die Einbauten freigelassenen Hohlräume und / oder die Medienleitungen mit ihren geringen Durchmessern strömen, um schließlich durch die schmalen und kleinen Öffnungen in die Umgebung auszutreten.

Ein an die Kupplungsvorrichtung angeschlossenes Instrument und der dadurch verringerte Durchmesser des Strömungswegs der Druckluft bewirken somit einen Rückstau in der Druckluftleitung, die die Druckluftquelle mit der Kupplungsvorrichtung verbindet, und einen Anstieg des in der Druckluftleitung anstehenden Luftdrucks (Fließdrucks). Ist an eine Kupplungsvorrichtung kein Instrument angeschlossen, so kann die Druckluft durch die vergleichsweise große Öffnung der Kupplungsvorrichtung und somit ohne großen Widerstand entweichen, so dass in diesem Fall kein oder nur ein geringer Rückstau in der Druckluftleitung und somit kein Anstieg des in der Druckluftleitung anstehenden Luftdrucks erfolgt. Das erfindungsgemäße Verfahren nutzt den Druckluftanstieg bei angeschlossenem Instrument, indem ein Sensorelement durch den in der Druckluftleitung anstehenden Luftdruck aktiviert wird und ein Eingangssignal an eine Steuervorrichtung des Pflege- oder Reinigungsgeräts sendet. Die Steuervorrichtung empfängt und verarbeitet das Eingangssignal und erkennt durch Auswertung des empfangenen Eingangssignals die Belegung der Kupplungsvorrichtung, d.h. ob sie mit einem Instrument belegt ist oder nicht.

In einem ersten Ausführungsbeispiel ist das Sensorelement als Sensor ausgebildet, wobei jeder bekannte Sensor verwendbar ist, bevorzugt kapazitive, induktive oder piezoelektrische Sensoren. Das durch den Sensor generierte Eingangssignal umfasst einen Messwert bzw. wird durch einen Messwert gebildet, der den in der Druckluftleitung anstehenden Luftdruck wiedergibt, der bei angeschlossenem Instrument dem erhöhtem Fließdruck entspricht, bei keinem angeschlossenen Instrument einem geringeren Druck. Ein Komparator der Steuervorrichtung vergleicht den empfangenen Messwert mit einem vorbestimmten Referenzwert und erkennt, dass die Kupplungsvorrichtung mit einem Instrument belegt ist, wenn der Messwert den Referenzwert überschreitet, und / oder dass die Kupplungsvorrichtung nicht belegt ist, wenn der Messwert den Referenzwert nicht überschreitet. Alternativ kann der Komparator durch Vergleich der Messwerte unterschiedliche Instrumententypen erkennen, insbesondere einen ersten Instrumententyp, der für die Druckluft leichter durchgängig ist, und / oder einen zweiten Instrumententyp, der zum Beispiel sehr gut abgedichtet ist, und damit einen höheren Rückstau der Druckluft verursacht.

Der Referenzwert ist in einem Speicher abgelegt und kann entweder ein unveränderbarer Fixwert sein, der bevorzugt bei der Herstellung des Pflege- oder Reinigungsgeräts durch den Hersteller festgelegt und abgespeichert wird. Alternativ ist der Referenzwert veränderbar und kann bevorzugt vom Anwender über eine Eingabevorrichtung, zum Beispiel einem Potentiometer, die mit der Steuervorrichtung und dem Speicher verbunden ist, verändert und abgespeichert werden. Somit besteht zum Beispiel die Möglichkeit mehrere unterschiedliche Instrumententypen zu erkennen und zu unterscheiden, insbesondere einen ersten Instrumententyp, der für die Druckluft leichter durchgängig ist als ein zweiter Instrumententyp, der sehr gut abgedichtet ist. Die Unterscheidung von Instrumententypen kann in vorteilhafter Weise dazu genützt werden, bei einem nachfolgenden Pflege- und/ oder Reinigungsverfahren die Instrumententypen unterschiedlich zu behandeln, zum Beispiel bestimmte Pflege- oder Reinigungsschritte nur einem der beiden Instrumententypen zukommen zu lassen. Insbesondere kann ein abschließender Pflegeschritt, bei dem Bauteile des Instruments mit Schmiermittel versorgt werden, weggelassen werden.

Ein veränderbarer Referenzwert kann auch erst bei der Messung selbst bestimmt werden, in dem zum Beispiel ein oder mehrere Messwerte des niedrigeren Druckniveaus, d.h. des Luftdrucks in jenen Leitungen, die mit Kupplungsvorrichtungen verbunden sind, an die kein Instrument angeschlossen ist, als Referenzwert verwendet wird, und alle Messwerte, die von diesem Referenzwert signifikant abweichen, d.h. diesen überschreiten, somit eine Kupplungsvorrichtung repräsentieren, an der ein Instrument angeschlossen ist.

Des Weiteren besteht die Möglichkeit anstatt einem Referenzwert zwei oder mehr Referenzwerte fix oder veränderbar festzulegen, so dass entweder zwei oder mehr Instrumententypen oder zwei oder mehr Instrumententypen und eine nicht belegte Kupplungsvorrichtung erkannt werden können. Neben den Speicherplätzen für die Referenzwerte können auch Speicherplätze für die Werte der gemessenen Luftdrücke vorhanden sein, so dass der Anwender diese Werte mittels einer Steuervorrichtung und mit ihr verbundenen Stellorganen abspeichern und ihnen jeweils spezifische Pflege- oder Reinigungsverfahren oder Verfahrensschritte, die in der Steuervorrichtung abgelegt sind, zuordnen kann. Damit schafft der Anwender ein Pflege- oder Reinigungsgerät, das auf jene Instrumententypen, die er verwendet, anpassbar ist. Bei einem folgenden Pflege- oder Reinigungsverfahren erkennt das Pflege- oder Reinigungsgerät den Instrumententyp anhand des Werts des Luftdrucks und wählt aufgrund dieses Werts das entsprechende Pflege- oder Reinigungsverfahren aus.

In einem zweiten Ausführungsbeispiel ist das Sensorelement als Schalter ausgebildet, wobei jeder bekannte Schalter, Mikroschalter oder integrierte elektronische Druckschalter, bevorzugt mechanische Druckschalter verwendbar ist. Das durch den Schalter generierte Eingangssignal umfasst ein Schaltsignal bzw. wird durch ein Schaltsignal gebildet, das der Schalter abgibt, wenn der in der Druckluftleitung anstehende Luftdruck den Schaltpunkt überschreitet. Der Schalter kann in bekannter Weise als Schließer oder Öffner (NO bzw. NC-Schalter) ausgeführt sein, so dass erfindungsgemäß unter dem Begriff "Schaltsignal" auch die Unterbrechung eines kontinuierlich anstehenden Signals bzw. das "Nicht-Erhalten eines Signals" verstanden wird.

Die mit dem Schalter verbundene Steuervorrichtung empfängt das Schaltsignal, verarbeitet es und erkennt, dass die Kupplungsvorrichtung mit einem Instrument belegt ist, wenn sie ein erstes Schaltsignal erhält, und / oder dass die Kupplungsvorrichtung nicht belegt ist, wenn sie ein zweites Schaltsignal erhält bzw. dass die Kupplungsvorrichtung mit einem ersten Instrumententyp belegt ist, wenn sie ein erstes Schaltsignal erhält, und / oder dass die Kupplungsvorrichtung mit einem zweiten Instrumententyp belegt ist, wenn sie ein zweites Schaltsignal erhält.

Es ist auch möglich, in einer Druckluftleitung mehrere Schalter mit unterschiedlichen Schaltpunkten anzuordnen, die so gewählt sind, dass sie jeweils bei einem bestimmten Luftdruck, der einem bestimmten Instrumententypen zuordenbar ist, ihre Schaltfunktion ausführen. Damit ist es möglich auch mit Schaltern Instrumententypen zu unterscheiden.

In einem weiteren Ausführungsbeispiel setzt die Steuervorrichtung weiters für jede Kupplungsvorrichtung eine Belegungsvariable derart und legt sie bevorzugt in einem Speicher ab, dass, wenn die Auswertung des empfangenen Eingangssignals des Sensorelements ergibt, dass die Kupplungsvorrichtung mit einem Instrument belegt ist, die Belegungsvariable einen ersten Zustand, und, wenn die Auswertung des empfangenen Eingangssignals ergibt, dass die Kupplungsvorrichtung nicht mit einem Instrument belegt ist, die Belegungsvariable einen zweiten Zustand annimmt. Entsprechend kann die Belegungsvariable einen ersten Zustand annehmen, wenn die Auswertung des empfangenen Eingangssignals ergibt, dass die Kupplungsvorrichtung mit einem ersten Instrumententyp belegt ist, und einen zweiten Zustand annehmen, wenn die Auswertung des empfangenen Eingangssignals ergibt, dass die Kupplungsvorrichtung mit einem zweiten Instrumententyp belegt ist. Unterscheidet die Steuervorrichtung zwei oder mehr Instrumententypen und eine nicht belegte Kupplungsvorrichtung oder mehrere Instrumententypen, so kann die Belegungsvariable selbstverständlich eine entsprechende Anzahl von n - Zuständen annehmen.

Das Setzen einer Belegungsvariablen ist insbesondere dann von Vorteil, wenn ein Pflege- oder Reinigungsgerät mehrere Kupplungsvorrichtungen hat und das Pflege- oder Reinigungsverfahren oder zumindest einzelne Schritte davon sequentiell ablaufen, d.h. für jedes Instrument oder für jeden Instrumententyp separat, und erst nach Beendigung des Verfahrens oder Verfahrensschritts für ein Instrument oder einen Instrumententyp das Verfahren oder der Verfahrensschritt für ein zweites Instrument oder einen zweiten Instrumententyp beginnt. Die Bestimmung an welcher Kupplungsvorrichtung ein Instrument angeschlossen ist oder nicht bzw. welche Instrumententypen angeschlossen sind, kann dann in vorteilhafter Weise vor dem eigentlichen Pflege- oder Reinigungsverfahren für alle Kupplungsvorrichtungen gemeinsam durchgeführt werden.

Nach Abschluss der Bestimmung ob ein Instrument an eine Kupplungsvorrichtung angeschlossen ist bzw. welcher Instrumententyp angeschlossen ist und vor dem Start des eigentlichen Pflege- oder Reinigungsverfahrens, schaltet die Steuervorrichtung eine ansteuerbare Pflege- oder Reinigungsmitteleinheit des Pflege- oder Reinigungsgerät bzw. Teile davon für jene Kupplungsvorrichtung frei, für die die Auswertung des Eingangssignals ergibt, dass sie mit einem Instrument belegt ist. Die Pflege- oder Reinigungsmitteleinheit ist über Leitungen mit jeder Kupplungsvorrichtung verbunden, so dass im folgenden Pflege-oder Reinigungsverfahren das an die Kupplungsvorrichtung angeschlossene Instrument entsprechend einem automatisch ablaufenden Programm der Steuervorrichtung mit Pflege-oder Reinigungsmitteln versorgt werden kann. Ist eine Kupplungsvorrichtung nicht mit einem Instrument belegt, so schaltet die Steuervorrichtung die ansteuerbare Pflege- oder Reinigungsmitteleinheit bzw. Teile davon nicht frei, so dass diese Kupplungsvorrichtung nicht mit Pflege- oder Reinigungsmitteln versorgt wird.

Ist die Kupplungsvorrichtung mit einem ersten und zweiten Instrumententyp belegt, so schaltet die Steuervorrichtung eine ansteuerbare Pflege- oder Reinigungsmitteleinheit des Pflege- oder Reinigungsgeräts bzw. Teile davon für alle Kupplungsvorrichtungen frei, wenn beide Instrumententypen mit einem Pflege- oder Reinigungsmittel versorgt werden sollen, bzw. gibt die Steuervorrichtung nur Kupplungsvorrichtungen mit jenen Instrumententypen frei, die mit einem Pflege- oder Reinigungsmittel versorgt werden sollen, das für den anderen Instrumententyp nicht vorgesehen ist. Entsprechend verfährt die Steuervorrichtung wenn die Belegungsvariable einen ersten, zweiten oder n-ten Zustand einnimmt, so dass für jeden Zustand die Pflege- oder Reinigungsmitteleinheit für eine Kupplungsvorrichtung freischaltbar ist oder nicht.

Die durch die Steuervorrichtung ansteuerbare Pflege- oder Reinigungsmitteleinheit umfasst bevorzugt eine oder mehrere ansteuerbare Sperrvorrichtungen, die die Verbindung zwischen der Pflege- oder Reinigungsmitteleinheit und der Kupplungsvorrichtung unterbrechen und absperren können. Dazu zählen bevorzugt Ventile, insbesondere Magnetventile, oder Schieber, die in einem Grundzustand geschlossen sind und durch die Ansteuerung geöffnet werden, oder Pumpen, die durch die Ansteuerung aktiviert werden und Medien fördern. Die selben ansteuerbaren Sperrvorrichtungen sind auch in den Luftleitungen zwischen der Luftquelle und den Kupplungsvorrichtungen vorhanden.

Die Erfindung wird nachfolgend anhand eines bevorzugten Ausführungsbeispiels und Bezug nehmend auf die beigefügte Zeichnung erläutert:

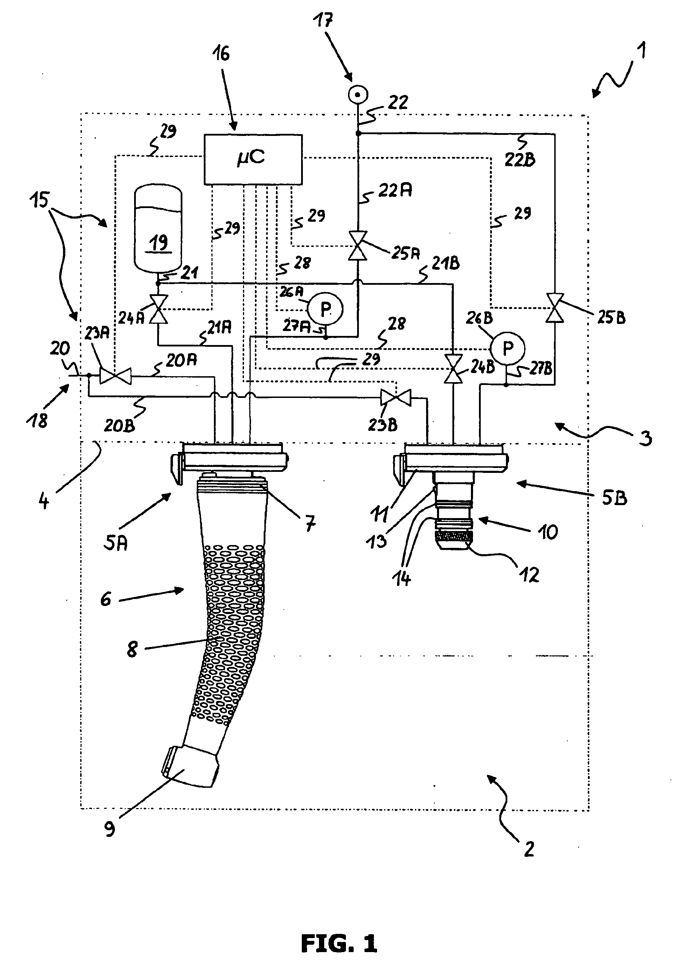

Figur 1 zeigt eine schematische Darstellung des erfindungsgemäßen Pflege- oder Reinigungsgeräts.

Das Pflege- oder Reinigungsgerät 1 besteht aus einem Pflegeraum 2 und zumindest einem weiteren Teilraum 3, die durch eine Wand 4 voneinander getrennt sind. Der Pflegeraum 2 ist von der Umgebung abgedichtet, so dass während eines Pflege- oder Reinigungsverfahrens keine Pflege- oder Reinigungsmedien nach außen austreten können. Durch eine verschließbare, zum Beispiel mit einer schwenkbaren oder verschiebbaren Tür versehene Öffnung können das oder die zu reinigenden Instrumente in den Pflegeraum 2 eingebracht werden. Im Pflegeraum 2 sind des Weiteren eine oder mehrere Kupplungsvorrichtungen 5A, 5B zum Anschluss des oder der zu reinigenden Instrumente 6 vorgesehen. Bei dem dargestellten Instrument 6 handelt es sich um ein dentales gewinkeltes Handstück, in dem mehrere Medienkanäle, zum Beispiel für Wasser und Druckluft, angeordnet sind und von der Kupplung 7 durch den Griffhülsenabschnitt 8 bis in oder in die Nähe des Handstückkopfes 9 verlaufen. Bei dem dargestellten dentalen gewinkelten Handstück handelt es sich jedoch nur um ein mögliches Instrument aus einer Vielzahl von medizinischen oder chirurgischen Instrumenten oder Instrumententeilen, etwa Handstückköpfen mit einem Halsabschnitt, die an die Kupplungsvorrichtung 5A, 5B anschließbar sind.

Die Kupplungsvorrichtung 5A, 5B kann grundsätzlich aus jeder bekannten Kupplungsvorrichtung gebildet sein, so zum Beispiel aus einer Schraub-, Steck-, Dreh- oder Schnellkupplung. Bevorzugt besteht sie jedoch aus einer Kupplung, die einem standardisierten Motoransatz 10 oder einer Turbinenhandstückkupplung ähnelt, so dass sich für den Anwender das Anschließen des zu reinigenden Instruments 6 nicht vom Anschluss des Instruments 6 an einen Antriebsmotor unterscheidet. Zum Anschluss von Instrumenten mit einem Anschlussbild, das nicht auf die Kupplungsvorrichtung 5A, 5B passt, können Adapter vorgesehen sein, die mit einem Ende lösbar mit der Kupplungsvorrichtung 5A, 5B und mit dem anderen Ende lösbar mit dem Instrument 6 verbindbar sind. Selbstverständlich können in einem Pflege- oder Reinigungsgerät 1 auch mehrere unterschiedliche Kupplungsvorrichtungen oder Kupplungsvorrichtungen mit unterschiedlichen Anschlussbildern vorgesehen sein.

Die einem standardisierten Motoransatz 10 ähnelnde Kupplung umfasst einen Anschlag 11, der in oder an der Wand 4 des Pflege- oder Reinigungsgeräts 1 befestigt ist und gleichzeitig die Einstecktiefe des Kupplungszapfens 12 in das Instrument 6 begrenzt. Der Kupplungszapfen 12 hat die selbe zylindrische Form und die selben Abmessungen wie der standardisierte Kupplungszapfen eines Motors und weist auch die gleiche Oberflächenstruktur auf. An der Oberfläche des Kupplungszapfens 12 münden zwei - bezogen auf die Längsachse des Kupplungszapfens 12 - radial angeordnete Bohrungen, die jeweils von zwei in Ringnuten angeordneten Dichtungen 14 umgeben sind. Über diese Bohrungen können Reinigungsmedien an die zu reinigenden Instrumente 6 abgegeben werden, wobei bevorzugt, wenn das Instrument 6 auf dem Kupplungszapfen 12 befestigt ist, jeweils ein Ringkanal in der Innenseite des Kupplungsrohrs des Instruments 6 über einer Bohrung angeordnet ist. Besonders bevorzugt ist einer der Ringkanäle mit einer Wasser führenden Leitung im Inneren des Instruments 6 und der zweite Ringkanal mit einer Druckluft führenden Leitung im Inneren des Instruments 6 verbunden, so dass diese Medienleitungen selektiv mit Reinigungs- und / oder Pflegemitteln versorgt werden können.

Der Kupplungszapfen 12 umfasst weiters eine federnde Rastnase 13, die, wenn das Instrument 6 auf der Kupplungsvorrichtung 5A, 5B befestigt ist, gegen die Innenseite des Kupplungsrohrs des Instruments 6 drückt und so das Instrument 6 auf dem Kupplungszapfen 12 zusätzlich fixiert.

Der Teilraum 3 des Pflege- oder Reinigungsgeräts 1 umfasst die Pflege- oder Reinigungsmitteleinheit 15, die Steuervorrichtung 16, einen Anschluss an eine Druckluftquelle 17 und einen Anschluss an eine Stromquelle (nicht dargestellt).

Je nach Art des Pflege- oder Reinigungsgeräts 1 umfasst die Pflege- oder Reinigungsmitteleinheit 15 einen Anschluss an eine Wasserquelle 18 und / oder an eine Quelle für ein Reinigungsmittel und / oder an eine Quelle für ein Desinfektionsmittel und /oder an einer Quelle für ein Schmiermittel. Die Quellen für das Reinigungsmittel, Desinfektionsmittel und Schmiermittel sind bevorzugt als Behälter ausgebildet, wobei in der schematischen Darstellung der Figur 1 symbolhaft für diese Quellen ein Behälter 19 abgebildet ist. Das erfindungsgemäße Pflege- oder Reinigungsgerät 1 kann jedoch einen oder mehrere Behältnisse für ein oder mehrere Reinigungs-, Desinfektions- und Schmiermittel umfassen. Der Anschluss an eine Wasserquelle 18 ist bevorzugt als Anschluss an eine Wasserleitung ausgebildet.

Wie aus Figur 1 zu erkennen ist, ist jede dieser Quellen mit jeder Kupplungsvorrichtung 5A, 5B durch eine eigene Leitung verbunden. Der Anschluss an eine Wasserquelle 18 ist über Leitung 20A mit Kupplungsvorrichtung 5A und über Leitung 20B mit Kupplungsvorrichtung 5B verbunden. Behälter 19 ist über Leitung 21A mit Kupplungsvorrichtung 5A und über Leitung 21 B mit Kupplungsvorrichtung 5B verbunden, genau so wie zusätzliche Behälter für weitere Pflege- oder Reinigungsmedien über jeweils separate Leitungen mit jeder Kupplungsvorrichtung 5A, 5B verbunden sein können. Die Leitungen 20A, 20B und 21A, 21 B zweigen jeweils von einer gemeinsamen Leitung 20, 21 ab.

Auch der Anschluss an eine Druckluftquelle 17 ist mit jeder Kupplungsvorrichtung 5A, 5B durch eine eigene Leitung 22A, 22B verbunden, die von einer gemeinsamen Leitung 22 abzweigen. Die Druckluftleitungen 22A, 22B setzen sich im Inneren der Kupplungen 5A, 5B in einer Leitung und / oder Bohrung fort, bevorzugt als zentrale Bohrung im Kupplungszapfen 12, die an einem Ende des Kupplungszapfen 12 über eine Öffnung in den Pflegeraum 2 mündet. In der Leitung 22 kann bevorzugt ein Druckregler vorgesehen sein, der es ermöglicht, dass das Pflege- oder Reinigungsgerät 1 an Druckluftquellen mit unterschiedlichem oder schwankendem Luftdruck angeschlossen werden kann, jedoch mit einem einheitlichen Luftdruck betrieben wird. Meist liegt der von unterschiedlichen Luftdruckquellen abgegebene Luftdruck in einem Bereich zwischen 4-10 bar, so dass bevorzugt ein Druckregler vorgesehen ist, der den Luftdruck auf einen Wert zwischen 2-4 bar senkt.

Umfasst das Pflege- oder Reinigungsgerät 1 weitere Kupplungsvorrichtungen, so ist in gleicher Weise jede Kupplungsvorrichtung über eine eigene Leitung mit den jeweiligen Quellen 17, 18, 19 verbunden. Selbstverständlich ist es auch möglich, dass die Leitungen 20A, 20B, 21 A, 21 B, 22A, 22B nicht von jeweils einer Hauptleitung 20, 21, 22 abzweigen, sondern dass sie direkt mit den jeweiligen Quellen 17, 18, 19 verbunden sind.

Die Pflege- oder Reinigungsmitteleinheit 15 umfasst mehrere ansteuerbare, als Ventile ausgebildete Sperrvorrichtungen 23A, 23B, 24A, 24B, die jeweils in den Leitungen 20A, 20B, 21A, 21 B angeordnet sind. Auch in den Luftleitungen 22A, 22B sind als Ventile, insbesondere als Magnetventile, ausgebildete Sperrvorrichtungen 25A, 25B vorgesehen. Die Sperrvorrichtungen 23A, 23B, 24A, 24B, 25A, 25B dienen dazu, die jeweilige Leitung 20A, 20B, 21A, 21 B, 22A, 22B für den Durchfluss des entsprechenden Mediums zu öffnen oder zu schließen. Jede dieser Sperrvorrichtung 23A, 23B, 24A, 24B, 25A, 25B ist mit der Steuervorrichtung 16 über eine Leitung 29 verbunden. Die Steuervorrichtung 16 ist als mechanisches Zeitschaltwerk, Schaltkreis oder Computer, besonders bevorzugt als Mikro-Controller, ausgeführt.

Jeder Kupplungsvorrichtung 5A, 5B ist des Weiteren ein im Teilraum 3 angeordnetes Sensorelement 26A, 26B, bevorzugt ein Sensor oder Schalter, zugeordnet. Jedes Sensorelement 26A, 26B ist direkt mit der Druckluftleitung 22A, 22B verbunden und insbesondere in der Druckluftleitung 22A, 22B oder in einer Abzweigung 27A, 27B der Druckluftleitung 22A, 22B angeordnet. Alternativ können die Sensorelemente 26A, 26B auf der Platine der Steuervorrichtung 16 angeordnet sein. Jedes Sensorelement 26A, 26B ist über eine Leitung 28 mit der Steuervorrichtung 16 verbunden. Die Sensorelemente 26A, 26B sind durch den in der jeweiligen Druckluftleitung 22A, 22B anstehenden bzw. ansteigenden Luftdruck aktivierbar, d.h. sie geben ein entsprechendes Eingangssignal über die Leitung 28 an die Steuervorrichtung 16 ab.

Im folgenden ist die Funktionsweise des Pflege- oder Reinigungsgeräts 1 in Zusammenhang mit einem Verfahren zur Bestimmung der Belegung der Kupplungsvorrichtungen 5A, 5B, d.h. ob eine oder mehrere der Kupplungsvorrichtungen 5A, 5B mit einem Instrument 6 belegt sind bzw. mit welchem Instrurüententyp sie belegt sind, beschrieben. Das Verfahren läuft vor dem Start des eigentlichen Pflege- oder Reinigungsverfahrens als eigenes Verfahren oder als ein Teilverfahren des Pflege- oder Reinigungsverfahrens ab, mit dem Ziel im anschließenden Pflege- oder Reinigungsverfahren nur jene Kupplungsvorrichtungen 5A, 5B mit Pflege- und Reinigungsmitteln zu versorgen, die mit einem Instrument 6 belegt sind bzw. wenn an die Kupplungsvorrichtungen 5A, 5B unterschiedliche Instrumententypen angeschlossen sind, diesen Instrumententypen unterschiedliche Pflege- oder Reinigungsverfahren oder Verfahrensschritte zukommen zu lassen.

Wird das Pflege- oder Reinigungsgerät 1, das an eine Wasserquelle und an eine Druckluftquelle angeschlossen ist, und dessen Quellen für Reinigungsmittel und / oder Desinfektionsmittel und / oder Schmiermittel gefüllt sind, eingeschaltet bzw. gestartet, so sind die Pflege- oder Reinigungsmitteleinheit 15 bzw. alle Sperrvorrichtungen 23A, 23B, 24A, 24B, 25A, 25B nicht freigeschaltet, d.h. geschlossen oder inaktiv, so dass kein Medium durch die entsprechenden Leitungen 20A, 20B, 21 A, 21 B, 22A, 22B gefördert werden kann. Der Anwender schließt das oder die zu reinigenden Instrumente 6 an die Kupplungsvorrichtungen 5A, 5B an und startet per Knopfdruck das Verfahren. Dazu sind am Pflege- oder Reinigungsgerät 1 in bekannter Weise ein oder mehrere Taster mit Schaltern vorgesehen, die mit der Steuervorrichtung 16 verbunden sind, so dass der Anwender ein oder mehrere in einem Speicher der Steuervorrichtung 16 abgelegte Betriebsprogramme für Pflege- oder Reinigungsverfahren starten, aus mehreren voreingestellten Betriebsprogrammen für Pflege- oder Reinigungsverfahren auswählen oder verschiedene Betriebsparameter von Pflege- oder Reinigungsverfahren verändern kann.

In einem ersten Schritt werden die Sperrvorrichtungen 25A, 25B der Druckluftleitungen 22A, 22B geöffnet, so dass Druckluft von der Druckluftquelle über den Anschluss 17, die Druckluftleitungen 22, 22A, 22B und durch die Kupplungsvorrichtung 5A, 5B fließen kann. Das Öffnen der Sperrvorrichtungen 25A, 25B kann wahlweise für jede Sperrvorrichtung 25A, 25B einzeln erfolgen, so dass das Verfahren sequentiell für jede Kupplungsvorrichtung 5A, 5B abläuft, oder für alle Sperrvorrichtung 25A, 25B gleichzeitig. Bei gleichzeitiger Ansteuerung muss durch geeignete Mittel, zum Beispiel Drosseln, sicher gestellt werden, dass sich die unterschiedlichen Druckverhältnisse in den Druckluftleitungen 22A, 22B nicht ausgleichen oder gegenseitig beeinflussen und die Messungen verfälschen. Ist an eine der Kupplungsvorrichtungen ein Instrument 6 angeschlossen, so wie es beispielhaft für Kupplungsvorrichtung 5A in Figur 1 dargestellt ist, so muss die Druckluft durch das Instrument 6 strömen, um an die Umgebung entweichen zu können. Aufgrund des verengten Strömungswegs durch das Instrument 6 entsteht ein Rückstau, der zu einem Anstieg des Luftdrucks (Fließdrucks) in der Leitung 22A führt. Die in Leitung 22B fließende Druckluft wird hingegen durch kein Instrument 6 beeinflusst, so dass sie ohne nennenswerten Widerstand und Rückstau in den Pflegeraum 2 entweichen kann. Beispielsweise beträgt der Staudruck der einströmenden Druckluft, nachdem sie den Druckregler in Leitung 22 passiert hat, etwa 2,5 bar und der Fließdruck in Leitung 22A durch das angeschlossene Instrument 6 etwa 2,0 bar. In Leitung 22B ohne angeschlossenem Instrument 6 beträgt der Fließdruck etwa 1,5 bar. Der Referenzwert, mit dem die beiden gemessenen Druckwerte verglichen werden, ist in diesem Beispiel bei etwa 1,7 bar festgelegt.

Die Sensorelemente 26A, 26B werden durch den in den jeweiligen Druckluftleitungen 22A, 22B anstehenden Luftdruck aktiviert und senden über die Leitungen 28 jeweils ein Eingangssignal an die Steuervorrichtung 16. Die Verarbeitung und Auswertung des Eingangssignals durch die Steuervorrichtung erfolgt, in Abhängigkeit, ob es sich bei dem Sensorelement 26A, 26B um einen Sensor oder einen Schalter handelt, in der bereits weiter oben dargestellten Art und Weise, so dass zur Vermeidung von Wiederholungen auf eine nochmalige Beschreibung verzichtet wird.

Die Bestimmung unterschiedlicher Instrumententypen, die an die Kupplungsvorrichtungen 5A, 5B angeschlossen sind, erfolgt in gleicher Weise, d.h. dass die unterschiedlichen Instrumententypen wiederum an der Veränderung des Luftdrucks in den jeweiligen Druckluftleitungen 22A, 22B und den dadurch generierten Eingangssignalen erkannt werden.

Nach der Bestimmung an welcher Kupplungsvorrichtung 5A, 5B ein Instrument 6 angeschlossen ist, steuert die Steuervorrichtung 16 jene Sperrvorrichtungen der Pflege- und Reinigungsmitteleinheit 15 und der Druckluftleitungen 22A, 22B an, die einer Kupplungsvorrichtung 5A, 5B zugeordnet sind, an der ein Instrument 6 angeschlossen ist, und öffnet bzw. aktiviert sie, so dass im darauf folgenden Pflege- oder Reinigungsverfahren Druckluft, Wasser sowie Pflege- und Reinigungsmittel nur zu diesen Kupplungsvorrichtungen gefördert wird. In der Figur 1 werden somit die Sperrvorrichtungen 23A, 24A, 25A geöffnet und die Sperrvorrichtungen 23B, 24B, 25B bleiben geschlossen.

Wurden durch das Verfahren verschiedene Instrumententypen bestimmt, so startet die Steuervorrichtung 16 automatisch oder auf Auswahl des Anwenders ein Pflege- oder Reinigungsverfahren, das bestimmte Verfahrensschritte nur für einen bestimmten Instrumententyp durchführt. So können zum Beispiel druckluftbetriebene Handstücke, so genannte Turbinenhandstücke, und elektrisch betriebene Zahnsteinentfernungshandstücke an die Kupplungsvorrichtungen 5A, 5B angeschlossen werden. Bei der Erkennung der Instrumententypen entweicht die Druckluft durch das Zahnsteinentfernungshandstück deutlich schwerer, so dass der Luftdruck in der Druckluftleitung, die über die Kupplungsvorrichtung 5A, 5B mit dem Zahnsteinentfernungshandstück verbunden ist, deutlich stärker ansteigt als in der Druckluftleitung, die mit dem Turbinenhandstück verbunden ist.

Bei dem darauf folgenden Pflege- oder Reinigungsverfahren wird zum Beispiel ein abschließender Pflegeschritt, bei dem Schmiermittel in die Instrumente gefördert wird, für die Zahnsteinentfernungshandstücke übersprungen, da in diesen Instrumenten keine bewegten Bauteile enthalten sind, die geschmiert werden müssen. Die Steuervorrichtung 16 belässt somit die Sperrvorrichtungen in den Leitungen, die Schmiermittel von einer Schmiermittelquelle zu den Kupplungsvorrichtungen 5A, 5B leiten, an die Zahnsteinentfernungshandstücke angeschlossen sind, geschlossen oder inaktiv und schaltet nur die übrigen Sperrvorrichtungen frei, d.h. öffnet oder aktiviert sie.

Alternativ besteht die Möglichkeit bei einem Pflege- und Reinigungsgerät mit Innen-und Außenreinigung an einem ersten Instrumententyp nur eine Außenreinigung und an einem zweiten Instrumententyp eine Außen- und Innenreinigung durchzuführen. Entsprechend werden bei jenem Instrumententyp, der nur außen gereinigt wird, durch die Steuervorrichtung nur Sperrvorrichtungen in jenen Medienleitungen freigeschalten, d.h. geöffnet oder aktiviert, die Medienquellen für die Außenreinigung mit Düsen im Pflegeraum verbinden.

Die Erfindung ist nicht auf das beschriebene Ausführungsbeispiel beschränkt, sondern umfaßt alle Ausführungsmöglichkeiten, die das prinzipielle, sinngemäße Funktionsprinzip der Erfindung nicht verändern.

## Patentansprüche

1. Verfahren zur Bestimmung ob eine Kupplungsvorrichtung (5A, 5B) eines Pflege- oder Reinigungsgeräts für medizinische Instrumente (1) mit einem Instrument (6) belegt ist bzw. mit welchem Instrumententyp sie belegt ist, **dadurch gekennzeichnet, dass**
über eine einer Kupplungsvorrichtung (5A, 5B) zugeordneten Druckluftleitung (22A, 22B) Druckluft durch die Kupplungsvorrichtung (5A, 5B) gefördert wird, ein Sensorelement (26A, 26B) durch den in der Druckluftleitung (22A, 22B) anstehenden Luftdruck aktiviert wird, so dass das Sensorelement (26A, 26B) ein Eingangssignal an eine Steuervorrichtung (16) des Pflege- oder Reinigungsgeräts (1) sendet und die Steuervorrichtung (16) durch Auswertung des empfangenen Eingangssignals die Belegung der Kupplungsvorrichtung (5A, 5B) bzw. den an die Kupplungsvorrichtung (5A, 5B) angeschlossenen Instrumenttyp erkennt.

2. Verfahren nach Anspruch 1, **dadurch gekennzeichnet, dass**
das Eingangssignal einen durch einen Sensor ermittelten Messwert umfasst, der den in der Druckluftleitung (22A, 22B) anstehenden Luftdruck wiedergibt, und die Steuervorrichtung (16) durch Vergleich des empfangenen Messwerts mit einem Referenzwert erkennt, dass die Kupplungsvorrichtung (5A, 5B) mit einem Instrument (6) belegt ist, wenn der Messwert den Referenzwert überschreitet, und / oder dass die Kupplungsvorrichtung (5A, 5B) nicht belegt ist, wenn der Messwert den Referenzwert nicht überschreitet bzw. dass die Kupplungsvorrichtung (5A, 5B) mit einem ersten Instrumententyp belegt ist, wenn der Messwert den Referenzwert überschreitet, und / oder dass die Kupplungsvorrichtung (5A, 5B) mit einem zweiten Instrumententyp belegt ist, wenn der Messwert den Referenzwert nicht überschreitet.

3. Verfahren nach Anspruch 1, **dadurch gekennzeichnet, dass**
das Eingangssignal ein Schaltsignal eines Schalters umfasst und die Steuervorrichtung (16) erkennt, dass die Kupplungsvorrichtung (5A, 5B) mit einem Instrument (6) belegt ist, wenn sie ein erstes Schaltsignal erhält, und / oder dass die Kupplungsvorrichtung (5A, 5B) nicht belegt ist, wenn sie ein zweites Schaltsignal erhält bzw. dass die Kupplungsvorrichtung (5A, 5B) mit einem ersten Instrumententyp belegt ist, wenn sie ein erstes Schaltsignal erhält, und / oder dass die Kupplungsvorrichtung (5A, 5B) mit einem zweiten Instrumententyp belegt ist, wenn sie ein zweites Schaltsignal erhält.

4. Verfahren nach einem der vorhergehenden Ansprüche, **dadurch gekennzeichnet, dass**
die Steuervorrichtung (16) des Weiteren für jede Kupplungsvorrichtung (5A, 5B) eine Belegungsvariable setzt und bevorzugt in einem Speicher ablegt, wobei, abhängig von der Auswertung des empfangenen Eingangssignals, die Belegungsvariable einen ersten, zweiten und gegebenenfalls n-ten Zustand annimmt, wenn die Kupplungsvorrichtung (5A, 5B) mit einem Instrument (6) verbunden oder nicht verbunden ist und / oder wenn ein erster, zweiter oder n-ter Instrumententyp erkannt wird.

5. Verfahren nach einem der vorhergehenden Ansprüche, **dadurch gekennzeichnet, dass**
die Steuervorrichtung (16) vor dem Start eines Pflege- oder Reinigungsverfahrens eine Pflege- oder Reinigungsmitteleinheit (15) des Pflege- oder Reinigungsgeräts (1) ansteuert und für jene Kupplungsvorrichtung (5A, 5B) freischaltet, für die die Auswertung des Eingangssignals ergibt, dass sie mit einem Instrument (6) belegt ist bzw. dass sie mit einem ersten Instrumententyp belegt ist bzw. dass die Ihr zugeordnete Belegungsvariable einen ersten, zweiten oder n-ten Zustand einnimmt, so dass Medien aus der Pflege- oder Reinigungsmitteleinheit (15) in das Instrument (6) förderbar sind.

6. Pflege- oder Reinigungsgerät für medizinische Instrumente (1) zur Durchführung eines Verfahrens nach einem der Ansprüche 1 - 5 mit einer oder mehreren Kupplungsvorrichtungen (5A, 5B) zum Anschluss eines oder mehrerer Instrumente (6), einem Anschluss an eine Druckluftquelle (17), einer Pflege- oder Reinigungsmitteleinheit (15) und einer Steuervorrichtung (16), wobei jeder Kupplungsvorrichtung (5A, 58) eine Druckluftleitung (22A, 22B), die die Kupplungsvorrichtung (5A, 5B) mit der Druckluftquelle verbindet, und ein Sensorelement (26A, 26B) zugeordnet ist wobei jedes Sensorelement (26A, 26B) mit der Steuervorrichtung (16) verbunden ist, und bei seiner Aktivierung ein Eingangssignal an die Steuervorrichtung (16) abgibt, so dass die Steuervorrichtung (16) durch Auswertung des Eingangssignals die Belegung der Kupplungsvorrichtung (5A, 5B) bzw. den an die Kupplungsvorrichtung (5A, 5B) angeschlossenen Instrumenttyp erkennt, **dadurch gekennzeichnet, dass**
das Sensorelement (26A, 268) durch den in der Druckluftleitung (22A, 22B) anstehenden Luftdruck aktivierbar ist.

7. Pflege- oder Reinigungsgerät (1) nach Anspruch 6, **dadurch gekennzeichnet, dass**
das Sensorelement (26A, 26B) durch einen Sensor, bevorzugt einen kapazitiven, induktiven oder piezo-elektrischen Sensor, oder durch einen Schalter, bevorzugt einen mechanischen Druckschalter, gebildet Ist.

8. Pflege- oder Reinigungsgerät (1) nach Anspruch 6 oder 7, **dadurch gekennzeichnet, dass**
das Sensorelement (26A, 26B) in einem vom Pflegeraum (2) getrennten Teilraum (3) des Pflege- oder Reinigungsgeräts (1) angeordnet ist.

9. Pflege- oder Reinigungsgerät (1) nach einem der Ansprüche 6 - 8, **dadurch gekennzeichnet, dass**
jedes Sensorelement (26A, 26B) direkt mit der Druckluftleitung (22A, 22B) verbunden ist und insbesondere in der Druckluftleitung (22A, 22B) oder in einer Abzweigung der Druckluftleitung (22A, 22B) angeordnet ist.

10. Pflege- oder Reinigungsgerät (1) nach einem der Ansprüche 6 - 8, **dadurch gekennzeichnet, dass**
die Steuervorrichtung (16) eine Platine umfasst, auf der zumindest ein Teil der Sensorelemente (26A, 26B) angeordnet ist.

11. Pflege- oder Reinigungsgerät (1) nach einem der vorhergehenden Ansprüche 6-10, **dadurch gekennzeichnet, dass**
die Pflege- oder Reinigungsmitteleinheit (15) mit der Steuervorrichtung (16) verbunden und durch sie ansteuerbar ist, so dass, wenn die Auswertung des Eingangssignals ergibt, dass eine Kupplungsvorrichtung (5A, 5B) mit einem Instrument (6) bzw. mit einem ersten Instrumententyp belegt ist, die Pflege- oder Reinigungsmitteleinheit (15) für diese Kupplungsvorrichtung (5A, 5B) durch die Steuervorrichtung (16) freischaltbar ist, wodurch Medien aus der Pflege- oder Reinigungsmitteleinheit (15) in das Instrument (6) förderbar sind.

## Claims

1. A process for determining whether a coupling device (5A, 5B) of a care or cleaning device for medical instruments (1) is occupied by an instrument (6) and/or by which type of instrument it is occupied,
**characterized in that**
compressed air is conveyed through a coupling device (5A, 5B) via a compressed air line (22A, 22B) allocated to a coupling device (5A, 5B), a sensor element (26A, 26B) is activated by the air pressure applied in the compressed air line (22A, 22B), so that the sensor element (26A, 26B) sends an input signal to a control device (16) of the care or cleaning device (1), and the control device (16) detects the occupancy of the coupling device (5A, 5B) and/or the type of instrument connected to the coupling device (5A, 5B) by analyzing the received input signal.

2. The process according to claim 1,
**characterized in that**
the input signal comprises a measured value determined by a sensor that reflects the air pressure applied in the compressed air line (22A, 22B), and by comparing the received measured value with a reference value, the control device (16) detects that the coupling device (5A, 5B) is occupied by an instrument (6) if the measured value exceeds the reference value and/or that the coupling device (5A, 5B) is not occupied if the measured value does not exceed the reference value and/or that the coupling device (5A, 5B) is occupied by a first type of instrument if the measured value exceeds the reference value and/or that the coupling device (5A, 5B) is occupied by a second type of instrument if the measured value does not exceed the reference value.

3. The process according to claim 1,
**characterized in that**
the input signal comprises a switching signal of a switch, and the control device (16) detects that the coupling device (5A, 5B) is occupied by an instrument (6) when it receives a first switching signal and/or that the coupling device (5A, 5B) is not occupied when it receives a second switching signal and/or that the coupling device (5A, 5B) is occupied by a first type of instrument when it receives a first switching signal and/or that the coupling device (5A, 5B) is occupied by a second type of instrument when it receives a second switching signal.

4. The process according to any one of the preceding claims,
**characterized in that**
the control device (16) further sets an occupancy variable for each coupling device (5A, 5B) and preferably stores it in a memory, whereby depending on the analysis of the received input signal, the occupancy variable assumes a first, second and optionally an n-th state when the coupling device (5A, 5B) is connected or not connected to an instrument (6) and/or when a first, second or n-th type of instrument is detected.

5. The process according to any one of the preceding claims, **characterized in that**
the control device (16) controls a care or cleaning agent unit (15) of the care or cleaning device (1) before the start of a care or cleaning process and releases it for the coupling device (5A, 5B) for which the analysis of the input signal reveals that it is occupied by an instrument (6) and/or that it is occupied by a first type of instrument and/or that the occupancy variable assigned to it assumes a first, second or n-th state, so that media can be conveyed from the care or cleaning agent unit (15) and into the instrument (6).

6. Care or cleaning device for medical instruments (1) for performing a process according to any one of claims 1 through 5, comprising one or more coupling devices (5A, 5B) for connecting one or more instruments (6), a connection to a compressed air source (17), a care or cleaning agent unit (15) and a control device (16), whereby each coupling device (5A, 5B) is assigned a compressed air line (22A, 22B) connecting the coupling device (5A, 5B) to the compressed air source, and a sensor element (26A, 26B), whereby each sensor element (26A, 26B) is connected to the control device (16), and delivers an input signal to the control device (16) when activated, so that by analyzing the input signal, the control device (16) detects the occupancy of the coupling device (5A, 5B) and/or the type of instrument connected to the coupling device (5A, 5B),
**characterized in that**
the sensor element (26A, 26B) can be activated by the air pressure applied in the compressed air line (22A, 22B).

7. The care or cleaning device (1) according to claim 6,
**characterized in that**
the sensor element (26A, 26B) is formed by a sensor, preferably a capacitive, inductive or piezoelectric sensor or by a switch, preferably a mechanical pressure switch.

8. The care or cleaning device (1) according to claim 6 or 7,
**characterized in that**
the sensor element (26A, 26B) is arranged in a partial space (3), which is separate from a care space (2) of the care or cleaning device (1).

9. The care or cleaning device (1) according to any one of claims 6 through 8,
**characterized in that**
each sensor element (26A, 26B) is connected directly to the compressed air line (22A, 22B) and in particular is arranged in the compressed air line (22A, 22B) or in a branch line of the compressed air line (22A, 22B).

10. The care or cleaning device (1),
**characterized in that**
the control device (16) comprises a circuitboard on which is arranged at least a part of the sensor elements (26A, 26B).

11. The care or cleaning device (1) according to any one of the preceding claims 6 through 10,
**characterized in that**
the care or cleaning agent unit (15) is connected to the control device (16) and can be controlled by it, so that when the analysis of the input signal reveals that a coupling device (5A, 5B) is occupied by an instrument (6) and/or by a first type of instrument, the care or cleaning agent unit (15) can be released by the control device (16) for this coupling device (5A, 5B), so that media can be conveyed from the care or cleaning agent unit (15) into the instrument (6).

## Revendications

1. Procédé de détermination pour savoir si un dispositif d'accouplement (5A, 5B) d'un appareil d'entretien et de nettoyage pour instruments médicaux (1) est équipé d'un instrument (6) ou avec quel type d'instrument il est équipé, **caractérisé en ce que** de l'air comprimé est transporté à travers un dispositif d'accouplement (5A, 5B) en passant par une conduite d'air comprimé (22A, 22B) attribuée à un dispositif d'accouplement (5A, 5B), un élément de détection (26A, 26B) est activé par la pression d'air apparaissant dans la conduite d'air comprimé (22A, 22B), si bien l'élément de détection (26A, 26B) émet un signal d'entrée vers le dispositif de commande (16) de l'appareil d'entretien et de nettoyage (1) et le dispositif de commande (16) reconnaît, par l'évaluation du signal d'entrée reçu, l'équipement du dispositif d'accouplement (5A, 5B) ou le type d'instrument qui est raccordé au dispositif d'accouplement (5A, 5B).

2. Procédé selon la revendication 1, **caractérisé en ce que**
le signal d'entrée comprend une valeur mesurée par un détecteur, qui donne la pression d'air apparaissant dans la conduite d'air comprimé (22A, 22B) et le dispositif de commande (16), par une comparaison entre la valeur mesurée reçue et une valeur de référence, reconnaît que le dispositif d'accouplement (5A, 5B) est équipé d'un instrument (6), lorsque la valeur mesurée dépasse la valeur de référence, et/ou que le dispositif d'accouplement (5A, 5B) n'est pas équipé, lorsque la valeur mesurée ne dépasse pas la valeur de référence, ou encore que le dispositif d'accouplement (5A, 5B) est équipé d'un premier type d'instrument, lorsque la valeur mesurée dépasse la valeur de référence, et/ou que le dispositif d'accouplement (5A, 5B) est équipé d'un deuxième type d'instrument lorsque la valeur mesurée ne dépasse pas la valeur de référence.

3. Procédé selon la revendication 1, **caractérisé en ce que**
le signal d'entrée comprend un signal de commutation d'un interrupteur et le dispositif de commande (16) reconnaît que le dispositif d'accouplement (5A, 5B) est équipé d'un instrument (6), lorsqu'il reçoit un premier signal de commutation, et/ou que le dispositif d'accouplement (5A, 5B) n'est pas équipé, lorsqu'il reçoit un deuxième signal de commutation, ou encore que le dispositif d'accouplement (5A, 5B) est équipé d'un premier type d'instrument, lorsqu'il reçoit un premier signal de commutation, et/ou que le dispositif d'accouplement (5A, 5B) est équipé d'un deuxième type d'instrument lorsqu'il reçoit un deuxième signal de commutation.

4. Procédé selon l'une des revendications précédentes, **caractérisé en ce que**
le dispositif de commande (16) définit en outre une variable d'équipement pour chacun des dispositifs d'accouplement (5A, 5B) et la dépose de préférence dans une mémoire, la variable d'équipement admettant un premier, un deuxième ou un nième état, en fonction de l'évaluation du signal d'entrée reçu, lorsque le dispositif d'accouplement (5A, 5B) est relié ou n'est pas relié à un instrument (6) et/ou lorsqu'un premier, un deuxième ou un nième type d'instrument est reconnu.

5. Procédé selon l'une des revendications précédentes, **caractérisé en ce que**
le dispositif de commande (16) active une unité de nettoyage ou d'entretien (15) de l'appareil d'entretien et de nettoyage (1) avant le commencement du procédé d'entretien ou de nettoyage, et libère le dispositif d'accouplement (5A, 5B) pour lequel l'évaluation indique qu'il est équipé d'un instrument (6) ou qu'il est équipé d'un premier type d'instrument ou encore que la variable d'équipement qui lui est attribuée admet un premier, un deuxième ou un nième état, dispositifs d'accouplement 5A, 5B façon à ce que des fluides puissent être transportés de l'unité de nettoyage ou d'entretien (15) vers l'instrument (6).

6. Appareil d'entretien et de nettoyage pour instruments médicaux (1), destiné à l'exécution d'un procédé selon l'une des revendications 1 à 5, avec un ou plusieurs dispositifs d'accouplement (5A, 5B) pour le raccordement d'un ou plusieurs instruments (6), un raccord à une source d'air comprimé (17), une unité de nettoyage ou d'entretien (15) et un dispositif de commande (16), une conduite d'air comprimé (22A, 22B) reliant le dispositif d'accouplement (5A, 5B) à la source d'air comprimé étant attribuée à chacun des dispositifs d'accouplement (5A, 5B), ainsi qu'un élément de détection (26A, 26B), chaque élément de détection (26A, 26B) étant relié au dispositif de commande (16), et émettant un signal d'entrée vers le dispositif de commande (16) lors de son activation, de sorte que par l'évaluation du signal d'entrée, le dispositif de commande (16) reconnaît l'équipement du dispositif d'accouplement (5A, 5B) ou le type d'instrument qui est raccordé au dispositif d'accouplement (5A, 5B), **caractérisé en ce que**
l'élément de détection (26A, 26B) peut être activé par la pression d'air apparaissant dans la conduite d'air comprimé (22A, 22B).

7. Appareil d'entretien et de nettoyage (1) selon la revendication 6, **caractérisé en ce que**
l'élément de détection (26A, 26B) est constitué d'un détecteur, de préférence un détecteur capacitif, inductif ou piézo-électrique, ou encore d'un interrupteur, de préférence un bouton poussoir mécanique.

8. Appareil d'entretien et de nettoyage (1) selon la revendication 6 ou 7, **caractérisé en ce que**
l'élément de détection (26A, 26B) est disposé dans une chambre partielle (3) de l'appareil d'entretien et de nettoyage (1), qui est séparée de la chambre de soin (2).

9. Appareil d'entretien et de nettoyage (1) selon l'une des revendications 6 à 8, **caractérisé en ce que**
chaque élément de détection (26A, 26B) est relié directement à la conduite d'air comprimé (22A, 22B) et se trouve notamment dans la conduite d'air comprimé (22A, 22B) ou dans un embranchement de la conduite d'air comprimé (22A, 22B).

10. Appareil d'entretien et de nettoyage (1) selon l'une des revendications 6 à 8, **caractérisé en ce que**
le dispositif de commande (16) comprend une platine sur laquelle est disposée au moins une partie des éléments de détection (26A, 26B).

11. Appareil d'entretien et de nettoyage (1) selon l'une des revendications 6 à 10, **caractérisé en ce que**
l'unité de nettoyage ou d'entretien (15) est reliée au dispositif de commande (16) et peut être activée par celui-ci, si bien que lorsque l'évaluation du signal d'entrée indique qu'un dispositif d'accouplement (5A, 5B) est équipé d'un instrument (6) ou d'un premier type d'instrument, l'unité de nettoyage ou d'entretien (15) peut être libérée par le dispositif de commande (16) pour ce dispositif d'accouplement (5A, 5B), ce qui permet de transporter des fluides de l'unité de nettoyage ou d'entretien (15) vers l'instrument (6).
